(19) Europäisches Patentamt
European Patent Office
Office européen des brevets

(11) Veröffentlichungsnummer: **0 024 293**
**B1**

(12) # EUROPÄISCHE PATENTSCHRIFT

(45) Veröffentlichungstag der Patentschrift: **02.03.83**

(51) Int. Cl.³: **C 07 D 307/08**, C 07 D 319/12, C 07 D 323/00

(21) Anmeldenummer: **80103902.5**

(22) Anmeldetag: **09.07.80**

(54) **Di- und/oder Oligomerisierung von cyclischen, aliphatischen Äthern und Verwendung der entstandenen Produkte als spezielle Lösungs- und Komplexierungsmittel.**

(30) Priorität: **24.07.79 DE 2929950**

(43) Veröffentlichungstag der Anmeldung: **04.03.81 Patentblatt 81/9**

(45) Bekanntmachung des Hinweises auf die Patenterteilung: **02.03.83 Patentblatt 83/9**

(84) Benannte Vertragsstaaten: **BE DE FR GB NL**

(56) Entgegenhaltungen:
**DE-A-2 525 697**
**DE-B-1 255 302**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Naarmann, Herbert, Dr., Haardtblick 15, D-6719 Wattenheim (DE)**
Erfinder: **Zdenek, Janosek, Dr., Université de Louvain, B-1348 Louvain La Neuve (BE)**
Erfinder: **Viehe, Heinz Guenter, Dr., Place L. Pasteur 1, B-1348 Louvain-La Neuve (BE)**
Erfinder: **Beaujean, Michel, Place Louis Pasteur, B-1348 Louvain-La Neuve (BE)**
Erfinder: **Merenyi, Robert, Place L. Pasteur 1, B-1348 Louvain-La Neuve (BE)**

**EP 0 024 293 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Di- und/oder Oligomerisierung von cyclischen, aliphatischen Äthern und Verwendung der entstandenen Produkte als spezielle Lösungs- und Komplexierungsmittel

Die Erfindung betrifft ein Verfahren zur Di- und/oder Oligomerisierung von cyclischen, aliphatischen Äthern und die Verwendung der erhaltenen Produkte als Lösungs- und Komplexierungsmittel.

Es ist bereits bekannt, durch oxidative Kupplung, z. B. von Isopropylgruppen enthaltenden aromatischen Verbindungen mit Hilfe von Sauerstoff oder Peroxiden Di- bzw. Oligomere herzustellen. Derartige Reaktionen sind in den Publikationen von V. V. Korshak et al in Polymer Science USSR 1 (1962), 925 bis 935 beschrieben. Ähnliche Systeme, aber immer auf Basis von aromatischen Isopropylderivaten, sind in den DE-AS 1 244 395, DE-AS 1 255 302, DE-OS 2 525 697 und DE-OS 2 050 009 erwähnt. Von L. Friedman und H. Shechter wird im Zusammenhang der Nonylamidmodifizierung von Olefinen die Reaktion von N,N'- Dimethyl- und N-Methylacetamid mit Di-tert.-butylperoxid erwähnt (vgl. Tetrahedron Letters 1961, 238 bis 242).

In der Literaturstelle Polymer Science USSR, Bd. 1 (1960), Seiten 341 bis 350 ist abgegeben, daß Isopropylgruppen enthaltende Benzole, Diisopropylketon, Diisopropylferrocen, Diphenyl, p-Xylol oder p-Dichlorbenzol mit Peroxiden zu Dimeren umgesetzt werden können. Bei der Umsetzung der Kohlenwasserstoffverbindungen mit Peroxiden ist keine Explosion zu erwarten gewesen, da lediglich aktive Wasserstoffatome an CH-Gruppen zur Reaktion gebracht werden.

Aufgabe der vorliegenden Patentanmeldung war es, ein Verfahren zur Herstellung von di- und/oder oligomeren Produkten von cyclischen, aliphatischen Äthern aufzufinden.

Eine weitere Aufgabe bestand darin, Lösungs- und Komplexierungsmittel auf der Basis von obigen cyclischen, aliphatischen Äthern aufzuzeigen, die nicht flüchtige Chelatisierungsmittel darstellen.

Diese Aufgaben wurden erfindungsgemäß durch ein Verfahren gelöst, bei dem cyclische, aliphatische Äther der allgemeinen Formeln (I) oder (II)

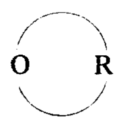

(I)

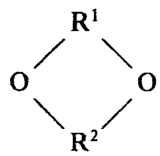

(II)

worin sein können

R = gesättigter, geradkettiger, aliphatischer $C_2$- bis $C_8$-Alkylenrest,

$R^1$ = gesättigter, geradkettiger, aliphatischer $C_2$- bis $C_4$-Alkylenrest und

$R^2$ = $R^1$ oder $(R^1-O)_n-R^1-$, wobei n = 1 bis 8

mit organischen Peroxiden bei Temperaturen von 120 bis 200°C in Schutzgasatmosphäre umsetzt.

Unter Di- und/oder Oligomerisierung obiger cycloaliphatischer Äther wird eine Reaktion verstanden, bei der zwei oder mehrere, maximal zehn Moleküle der gleichen Verbindung zusammengelagert werden. Diese Reaktion ist an sich bekannt und braucht hier nicht weiter erläutert zu werden.

Auch die gemäß Erfindung einzusetzenden Verbindungen der allgemeinen Formeln (I) oder (II) sind an sich bekannt.

Als erfindungsgemäße cyclische, aliphatische Äther der Formeln (I) oder (II) kommen insbesondere die Verbindungen (III) bis (V) in Frage:

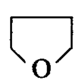

Tetrahydrofuran (III)

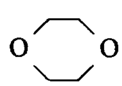

Dioxan (IV)

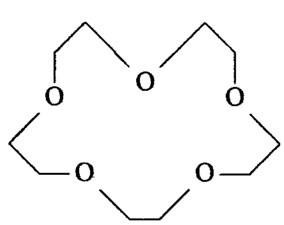

5-Kronen-15-äther (V)

Zur Durchführung der Dimerisierungs- bzw. Oligomerisierungsreaktion werden die cycloaliphatischen Äther der allgemeinen Formeln (I) oder (II) mit einem organischen Peroxid, vorzugsweise mit Di-tert.-butylperoxid, im molaren Verhältnis 0,1 : 1 bis 1 : 0,1, vorzugsweise im molaren Verhältnis 1 : 1, bei Temperaturen zwischen 120 und 200, vorzugsweise 150 und 180°C, erhitzt. Als Schutzatmosphäre dient ein Inertgas, vorzugsweise Stickstoff. Die erfindungsgemäßen Produkte werden durch fraktionierte Destillation bzw. Umkristallisation gereinigt. Die erhaltenen Di- und/oder Oligomerisierungsprodukte der Verbindungen III bis V weisen die folgenden Strukturformeln auf:

Verbindung III ⟶ 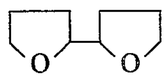 $kp_{266-400\ Pascal}$ 40–45°C

Verbindung IV ⟶ 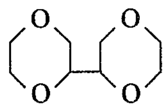 $kp_{266-400\ Pascal}$ 52–53°C

Verbindung V ⟶ 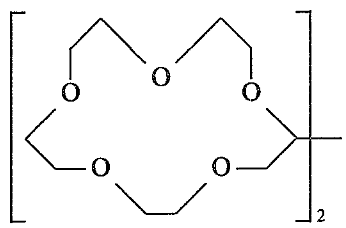 $kp_{6,65\ Pascal}$ 106–115°C

+ 10 bis 20 Gew.-% oligomere Produkte.

Der mit der Erfindung erzielte Vorteil liegt insbesondere darin, daß nicht flüchtige Chelatisierungsmittel zur Verfügung gestellt werden.

In den nachfolgenden Beispielen 1 bis 3 wird die Versuchsdurchführung erläutert. Die in den Beispielen genannten Teile und Prozente beziehen sich auf das Gewicht.

### Beispiel 1

Dimerisierung von Verbindung III

20 Teile (III) und 1 Teil Di-tert.-butylperoxid werden unter Stickstoff im Autoklaven 8 Stunden lang auf 160°C erhitzt. Nach dem Abkühlen wird das Dimerisierungsprodukt von (III) durch fraktionierte Destillation (40 bis 45°C, 266–400 Pascal) abgetrennt. Es werden 79% Dimerisierungsprodukt erhalten.

### Beispiel 2

Dimerisierung von Verbindung IV

20 Teile (IV) und 1 Teil Di-tert.-butylperoxid werden für 8 Stunden im Autoklaven unter Stickstoff auf 160°C erhitzt. Nach der Destillation (266–400 Pascal, 52–53°C) werden 72% eines öligen Dimeren erhalten.

### Beispiel 3

Dimerisierung von Verbindung V

20 Teile (V) und 1 Teil Di-tert.-butylperoxid werden im Autoklaven 8 Stunden lang unter Stickstoff auf 160°C erhitzt. Nach dem Abkühlen wird das Dimerisierungsprodukt bei 6,65 Pascal und 106–115°C fraktioniert. Es werden 81% Dimerisierungsprodukt erhalten.

## Patentansprüche

1. Verfahren zur Di- und/oder Oligomerisierung von cyclischen aliphatischen Äthern, dadurch gekennzeichnet, daß man Verbindungen der allgemeinen Formel (I) oder (II):

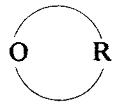

(I)

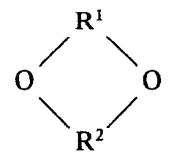

(II)

worin sein können

R = gesättigter, geradkettiger, aliphatischer $C_2$- bis $C_8$-Alkylenrest,

$R^1$ = gesättigter, geradkettiger, aliphatischer $C_2$- bis $C_4$-Alkylenrest und

$R^2$ = $R^1$ oder $(R^1—O)_n—R^1—$, wobei n = 1 bis 8

mit organischen Peroxiden bei Temperaturen von 120 bis 200°C in Schutzgasatmosphäre umsetzt.

2. Verwendung der nach Anspruch 1 hergestellten Di- und/oder Oligomerisierungsprodukte von cyclischen, aliphatischen Äthern als spezielle Lösungs- und Komplexierungsmittel.

**Claims**

1. A process for the dimerization and/or oligomerization of cyclic, aliphatic ethers, wherein a compound of the general formula (I) or (II)

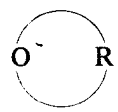

(I)

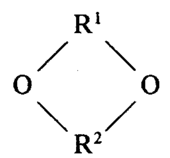

(II)

where

R is saturated, straight-chain, aliphatic $C_2$–$C_8$-alkylene,

$R^1$ is saturated, straight-chain, aliphatic $C_2$–$C_4$-alkylene and

$R^2$ is $R^1$ or $(R^1—O)_n—R^1—$, where n is from 1 to 8,

is reacted with an organic peroxide at from 120 to 200°C in a protective gas atmosphere.

2. Use of the dimerization and/or oligomerization products of cyclic, aliphatic ethers, obtained according to claim 1, as special solvents and complexing agents.

**Revendications**

1. Procédé de dimérisation et(ou) d'oligomérisation d'éthers aliphatiques cycliques, caractérisé en ce que l'on fait réagir des composés de l'une des formules générales (I) et (II)

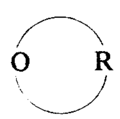

(I)

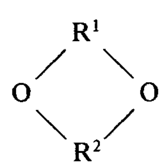

(II)

dans lesquelles

R désigne un groupe alcoylène aliphatique saturé à chaîne droite en $C_2$à $C_8$;

$R^1$ représente un groupe alcoylène aliphatique saturé à chaîne droite en $C_2$ à $C_4$ et
$R^2$ possède la même signification que $R^1$ ou est un groupe de la formule $(R^1—O)_n—R^1—$, où n vaut 1 à 8,

sous atmosphère d'un gaz protecteur et à des températures de 120 à 200°C avec des peroxydes organiques.

2. Utilisation des produits de dimérisation et(ou) d'oligomérisation d'éthers aliphatiques cycliques, préparés par le procédé de la revendication 1, comme solvants et complexants spéciaux.